# EUROPEAN PATENT APPLICATION

(11) **EP 4 502 569 A1**
(43) Date of publication of application: **05.02.2025**
(21) Application number: 23780811.8
(22) Date of filing: 30.03.2023
(51) Int. Cl.: G01N 1/10, G01N 1/00

(54) **MICROCHANNEL DEVICE**

(30) Priority: 30.03.2022 JP 2022055919
(71) Applicant: Rigaku Corporation, Akishima-shi Tokyo 196-8666 (JP)
(72) Inventor: SATO, Takashi, Akishima-shi, Tokyo 196-8666 (JP); MATSUMOTO, Takashi, Akishima-shi, Tokyo 196-8666 (JP); HASEGAWA, Tomokazu, Akishima-shi, Tokyo 196-8666 (JP)
(74) Representative: Lambacher, Michael
(86) International application number: PCT/JP2023/013048
(87) International publication number: WO 2023/190803

(57) **Abstract**

This microchannel device separates a sample solution, which contains a first component to be used as an analysis control and a second component to be analyzed, into a solution containing the first component and a solution containing the second component.

## Description

### TECHNICAL FIELD

The present invention relates to a microchannel device. More specifically, the present invention relates to a microchannel device for separation into a biological macromolecule and other components contained in a sample.

### BACKGROUND ART

Patent Document 1 discloses a microfluidic device having a mechanism for filtering a blood sample containing blood components. The microchannel device is characterized in that it is constituted of: an aperture for introducing the blood sample; an inclined type membrane filter disposed in a lower part side of the opening part; a support film disposed under the inclined type membrane filter for holding it; a space disposed under the support film for storing filtered blood components; a flow channel connected to the space; and an outlet part disposed at the opposite side of the space and connected to the flow channel, wherein the inclined type membrane filter has a distribution of filtration diameters of the filter gradually getting smaller from the opening part side toward the space side, and wherein the support film has through holes having a diameter of 1 µm to 500 µm with an aperture ratio of 5% to 60%.

### PRIOR ART DOCUMENTS

### PATENT DOCUMENTS

Patent Document 1: Japanese Laid-Open Patent Publication No. 2008-76306

### SUMMARY OF THE INVENTION

### PROBLEMS TO BE SOLVED BY THE INVENTION

Conventionally, when measuring a sample containing a biomacromolecule, a comparative sample (control) containing a component other than the biopolymer is prepared in addition to the sample, and the structure, etc. of the biopolymer are specified by comparison between the measurement value of the sample and the measurement value of the control.

However, actually, since the volume of the biopolymer is not taken into consideration with respect to the separately prepared control, the concentration of the component other than the biopolymer in the control is lower than the concentration of the component in the sample containing the biopolymer. Further, in the case where the biopolymer is derived from a cell, it is substantially impossible to specify an excreted substance from the cell. Therefore, in the case where the biopolymer contained in the sample is measured by taking the difference between the measurement value of the sample and the measurement value of the control, to be exact, the difference does not accurately reflect the biopolymer. Moreover, since it is impossible to subject the target (sample) to subtraction and correction using the control (comparative sample) with high resolution, it is difficult to realize analysis requiring high resolution such as calculation of the electron density of the target using small-angle X-ray scattering and structure analysis of the target using analytical ultracentrifugation.

In consideration of the above circumstances, the present invention provides a microchannel device for separation into a biopolymer as a target in a sample and components other than the biopolymer in the sample.

### MEANS FOR SOLVING THE PROBLEMS

The present invention includes aspects described below.
[1] A microchannel device that separates a sample solution containing a first component and a second component having a larger size than the first component, into a solution containing the second component and a solution containing the first component but not the second component, and the microchannel device has
a filter (first filter) with a pore size that allows the first component to pass through but does not allow the second component to pass through;
a first flow channel connected to a first reservoir section for storing the solution that passes through the first filter; and
a second flow channel connected to a second reservoir section for storing the solution that does not pass through the first filter.

[2] The device according to item [1], wherein the first component is a component used as an analysis control and the second component is a component to be analyzed.
[3] The device according to item [1], wherein the first component is a component to be analyzed and the second component is a component used as an analysis control.
[4] The microchannel device according to item [1], wherein the sample solution further contains a third component which is a component other than the first component and the second component and has a size larger than those of the first component and the second component, and wherein the microchannel device separates the sample solution into a solution containing the third component and a solution containing the first component and the second component but not containing the third component, the microchannel device further comprising:
a filter (second filter) having a pore size that allows the first component and the second component to pass through but does not allow the third component to pass through; and
a third flow channel connected to a third storage section for storing a solution that does not pass through the second filter,
wherein the second filter is arranged between the second flow channel and the third flow channel.

[5] The device according to item [4], wherein the first component, the second component and the third component are components combined according to any one of (a) to (f) below:

**Table 1**

| | First component | Second component | Third component |
|---|---|---|---|
| (a) | Component to be used as analysis control | Component to be analyzed | Component other than the first component and the second component |
| (b) | Component to be analyzed | Component to be used as analysis control | Component other than the first component and the second component |
| (c) | Component to be used as analysis control | Component other than the first component and the third component | Component to be analyzed |
| (d) | Component other than the second component and the third component | Component to be used as analysis control | Component to be analyzed |
| (e) | Component to be analyzed | Component other than the first component and the third component | Component to be used as analysis control |
| (f) | Component other than the second component and the third component | Component to be analyzed | Component to be used as analysis control |

[6] The microchannel device according to item [1], which further comprises a circulation flow channel (first circulation flow path) that is connected to the first filter and stores and circulates the sample solution containing the first component and the second component.
[7] The microchannel device according to item [4], which further comprises a circulation flow channel (second circulation flow channel) that is connected to the second filter and stores and circulates the sample solution containing the first component, the second component and the third component.
[8] The microchannel device according to item [1], wherein the second component consists of a biopolymer and the first component consists of a low-molecular-weight substance which prepare an environment of the biopolymer.
[9] The microchannel device according to item [4], wherein the second component consists of a biopolymer, the first component consists of a low-molecular-weight substance which adjusts an environment of the biopolymer, and the third component is an aggregate, a cell residue or a coarse particle.
[10] The microchannel device according to item [4], wherein the second filter has pores having a size that is smaller than that of the third component and larger than those of the first component and the second component.
[11] A method for flowing a sample solution through the microchannel device according to item [1] to separate the sample solution into a solution containing the second component and a solution containing the first component but not containing the second component.
[12] A method for flowing a sample solution through the microchannel device according to item [4] to separate the sample solution into a solution containing the third component and a solution containing the first component and the second component but not containing the third component, followed by separating into a solution containing the second component and a solution containing the first component but not containing the second component.

### BRIEF DESCRIPTION OF THE DRAWINGS

FIG. 1 shows conceptual diagrams of the microchannel device of the present invention.
FIG. 2 shows the flow of each component with respect to the first filter, and the flow of each component with respect to the first filter and the second filter.
FIG. 3 is a schematic configuration diagram of a microchannel device 100 according to one embodiment of the present invention.
FIG. 4 shows schematic configuration diagrams of the second filter portion.
FIG. 5 shows schematic configuration diagrams of the first filter portion.
FIG. 6 shows graphs showing results obtained by separation into a biopolymer as a target and a control component using a microchannel device according to one embodiment of the present invention and measurement of X-ray diffraction data of both of them.

### BEST MODE FOR CARRYING OUT THE INVENTION

The present invention relates to a microchannel device that separates a sample solution, which contains a first component and a second component having a size larger than that of the first component, into a solution containing the second component and a solution containing the first component but not containing the second component (aspect 1).

Further, the present invention provides a microchannel device that separates a sample solution, which contains a first component, a second component and a third component that is a component other than the first component and the second component, into a solution containing the third component and a solution containing the first component and the second component but not containing the third component, followed by separating into a solution containing the second component and a solution containing the first component but not containing the second component (aspect 2).

The above-described aspect 1 relates to a microchannel device that separates respective components from each other when the size of each component increases in the order of the first component and the second component (first component < second component).

The above-described aspect 2 relates to a microchannel device that separates respective components from each other when the size of each component increases in the order of the first component, the second component and the third component (first component < second component < third component).

### <Regarding Aspect 1>

The microchannel device of the present invention has: a filter (first filter) having pores with a size that allows the first component to pass through but does not allow the second component to pass through; a first flow channel connected to a first storage section for storing a solution that passes through the first filter; and a second flow channel connected to a second storage section for storing a solution that does not pass through the first filter.

In this specification, a "component" refers to both a single component and an assembly of a plurality of components (Multi-component).

The microchannel device of the present invention has: a first filter that allows a solution containing the first component to pass through but does not allow the second component to pass through; a first flow channel, through which a solution that passes through the first filter (containing the first component but not containing the second component) is passed; and a second flow channel, through which a solution that does not pass through the first filter (containing the first component and the second component) is passed. The first flow channel is connected to a first storage section, and the second flow channel is connected to a second storage section for storing a solution that does not pass through the second filter.

Further, the second component has a size that is larger than that of the first component. For example, the second component has a molecular weight that is higher than that of the first component.

In the present invention, when measuring a sample containing a substance to be analyzed (e.g., biopolymer), it is not required to prepare a comparative sample (control) containing a component other than the biopolymer in addition to the sample, and it is possible to separate a sample solution containing the substance to be analyzed into a solution containing the component to be analyzed and a solution not containing the component to be analyzed and to use the solution not containing the component to be analyzed as a control. Since one of solutions contained in the same sample is used as a measurement target and the other is used as a control, the measurement target can be accurately normalized by subtracting the value of the control from the measurement result.

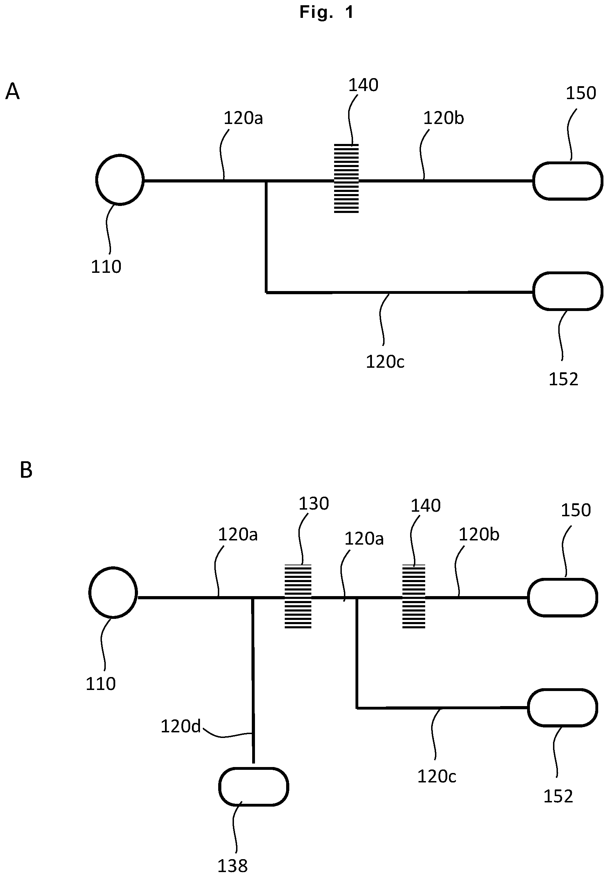

Conceptual diagrams of the microchannel device of the present invention are shown in Figure 1.

In Figure 1, A has: a sample solution inlet (sample inlet) 110; a sample solution flow channel 120a; a first filter 140 which allows the first component to pass through but does not allow the second component having a size larger than that of the first component to pass through; a flow channel 120b for a solution containing the first component, which is connected to a storage section (first storage section 150) for storing the solution containing the first component; and a flow channel 120c for a solution containing the second component, which is connected to a storage section (second storage section 152) for storing the solution containing the second component (this solution may contain the first component). Note that the first storage section 150 and the second storage section 152 may be arranged inside or outside the microchannel device of the present invention.

The sample inlet 110 is an opening for introducing the sample solution containing the first component and the second component into the flow channel 120a. The sample inlet 110 and the first filter 140 are connected by the flow channel 120a. Further, the flow channel 120a has a flow channel 120c that branches off at a position between the sample inlet 110 and the first filter 140. The flow channel 120c is connected to the second storage section 152 for storing the second component. In this specification, when the entire flow channels such as the flow channels 120a, 120b and 120c are collectively described, they are sometimes simply referred to as "flow channel 120".

In the present invention, examples of the combination of the first component and the second component include: (i) a combination in which the first component is a component to be used as an analysis control (also referred to as a "control component") and the second component is a target component for analysis (also referred to as a "component to be analyzed"); and (ii) a combination in which the first component is a component to be analyzed and the second component is a control component.

### (i) Combination in which the first component is control component and the second component is component to be analyzed

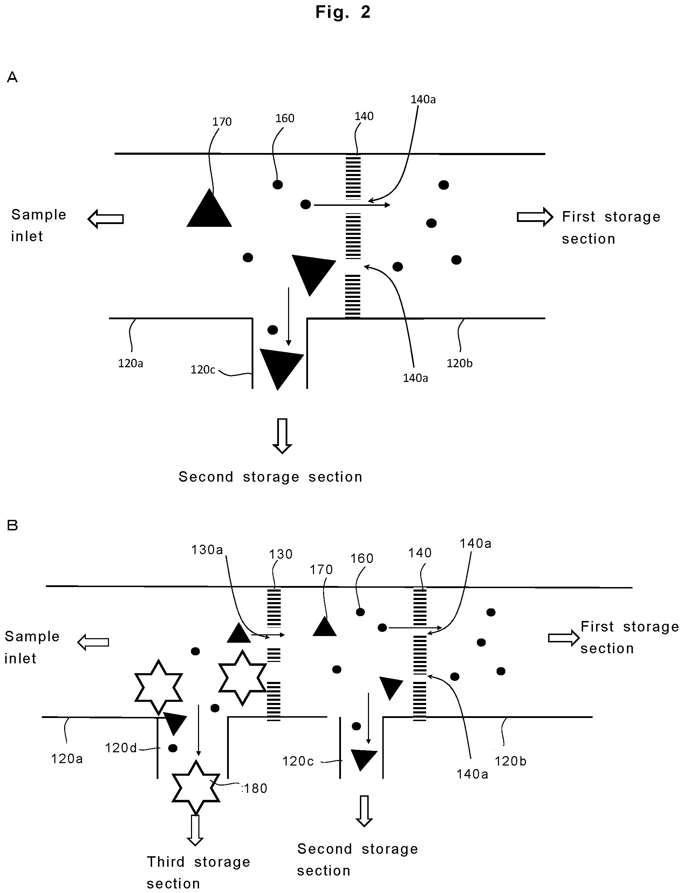

Figure 2A shows an way in which a solution containing a first component 160 and a solution containing a second component 170 flow through the flow channels in Figure 1A. Figure 2Ais a schematic diagram of an aspect in which a solution containing the second component 170 and a solution containing the first component 160 but not containing the second component 170 are separated via the first filter 140. In this case, the first component 160 is a control component, and the second component 170 is a component to be analyzed having a size that is larger than that of the first component 160 as the control component.

Between the sample inlet 110 and the first filter 140, a sample solution containing the first component 160 and the second component 170 is flowed. In the first filter 140, a pore 140a having a size that allows the first component 160 to pass through but does not allow the second component 170 to pass through is formed, and therefore the first component 160 can pass through the pore 140a of the first filter 140. Meanwhile, the second component 170 is a molecule larger than the first component 160 and is larger than the pore 140a, and therefore cannot pass through the pore. Further, the diameter of the flow channel 120c is larger than the second component 170. Accordingly, the second component 170 flows into the flow channel 120c connected to the second storage section 152.

In this regard, the first component 160 passes through the first filter 140, but there is a case where the first component 160 does not pass through the first filter 140 but passes through the flow channel 120c and flows into the storage section 152. Further, there is a case where the first component 160 passes through the first filter 140, then is returned to the inlet 110 or flow channel 120a via a recirculation flow path (not shown), and passes through the first filter 140 again, and there is a case where the first component 160 passes through the first filter 140 and then reverse to the pore 140a. In other words, the first component 160 is in a state where it can move through the pore 140a of the first filter 140 in both left and right directions. Meanwhile, the second component 170 cannot pass through the first filter 140, and passes through the flow channel 120c and flows into the storage section 152 together with the first component 160. The solution that flows through the flow channel 120b is a solution containing the first component 160 but not containing the second component 170, and the solution that flows through the flow channel 120c is a solution containing the first component 160 and the second component 170. That is, a solution containing the first component 160 can fill the flow channel 120b, the storage section 150, the flow channel 120c and the storage section 152, and a solution containing the first component 160 and the second component 170 (biopolymer) can fill the flow channel 120c and the storage section 152. In the present invention, a flow channel (not shown) connected to the sample inlet 110 may be provided to recirculate a solution that flows into the storage section 152 from the storage section 152.

In the case of the combination in which the first component is a component to be used as an analysis control and the second component is a component to be analyzed, the first component 160 is a group of low-molecular-weight molecules which adjusts an environment of the second component 170 (biological macromolecule, etc) to be analyzed, and the first component 160 includes various buffering agents and stabilizers. Examples of buffering agents include various buffer components such as a phosphate buffer solution, a citrate buffer solution, a borate buffer solution, and a HEPES buffer solution. Stabilizers are components to beadded for maintaining the quality and safety of a component to be analyzed, and examples thereof include preservatives, antioxidants, chelating agents, pH adjusters, a thickeners, and dispersants. Further, according to the purpose of analysis, a polysaccharide, a nucleic acid, a synthetic polymer, etc. can also be employed as stabilizers.

Since a solution that passes through the first filter 140 from the sample solution containing the first component 160 and the second component 170 does not contain the second component 170, the solution that passes through the first filter 140 is used as a control solution for analysis of the second component.

### (ii) Combination in which the first component is component to be analyzed and the second component is component to be used as analysis control

Further, the present invention includes an aspect of a combination in which the first component is a component to be analyzed and the second component is a component to be used as an analysis control. In this case, the first component is a component to be analyzed having a size smaller than that of a control component and the second component is the control component. In this aspect, the first component as the component to be analyzed is a biopolymer, and examples of the second component as the control component include a polysaccharide, a nucleic acid, and a synthetic polymer.

### <Regarding Aspect 2>

In another aspect of the present invention, in the case where the sample solution further contains, in addition to the first component and the second component, a third component which is a component other than the first component and the second component, the present invention provides a microchannel device for separation into a solution containing the third component and a solution containing the first component and the second component but not containing the third component. In this aspect, the microchannel device of the present invention has: a filter (second filter) having pores with a size that allows the first component and the second component to pass through but does not allow the third component to pass through; and a third flow channel connected to a third storage section for storing a solution that does not pass through the second filter, and the second filter is arranged between the second flow channel and the third flow channel.

Figure 1B has, in addition to the constitution of Figure 1A, a second filter 130 which allows a solution containing the first component 160 and the second component 170 to pass through but does not allow a solution containing a component other than the first component 160 and the second component 170 (this is referred to as the third component) to pass through, and the second filter 130 is connected between the sample inlet 110 and the first filter 140 via the flow channel 120a.

In Figure 1B, the sample inlet 110 is an opening for introducing the sample solution containing the first component 160, the second component 170 and the third component 180 (see Figure 2B) into the flow channel 120. The sample inlet 110 and the second filter 130 are connected by the flow channel 120a. Further, the microchannel device of the present invention has a flow channel 120d that branches from the flow channel 120a at a position between the sample inlet 110 and the second filter 130. The flow channel 120d is connected to a third storage section 138 for storing the third component.

In the case where a sample solution containing the third component that is a component other than the first component 160 and the second component 170 is flowed, the microchannel device having the second filter 130 is used. In this case, the second filter 130 is arranged between the sample inlet 110 and the first filter 140.

Figure 2B shows an aspect in which a solution containing the first component 160, the second component 170 and the third component 180 flows through the flow channels in Figure 1B. Figure 2B is a schematic diagram of an aspect in which a solution containing the third component 180 and a solution containing the first component 160 and the second component 170 but not containing the third component 180 are separated via the second filter 130. In this case, the size of each component increases in the order of the first component 160, the second component 170 and the third component 180.

In the second filter 130, a pore 130a having a size that allows the first component 160 and the second component 170 to pass through but does not allow the third component 180 to pass through is formed, and therefore the first component 160 and the second component 170 can pass through the pore 130a of the second filter 130. Meanwhile, the third component 180 cannot pass through the pore 130a of the second filter 130. Further, the diameter of the flow channel 120d is larger than the third component 180. Accordingly, the third component 180 does not pass through the second filter 130 and flows into the flow channel 120d connected to the third storage section 138.

In this regard, in the flow channel 120d, the third component 180 flows, and the first component 160 and the second component 170, which have a size smaller than that of the third component 180, may also flow. A solution that flows through the flow channel 120d and is stored in the third storage section can be discarded, but the solution can also be reused by providing a flow channel (not shown) for returning the solution to the sample inlet 110. Further, in the case of a sample solution containing a plurality of types of components such as the first component 160 and the second component 170 which pass through the second filter 130 and the third component 180 which does not pass through the second filter 130, the third component 180 which does not pass through the second filter 130 can also be used as a measurement target.

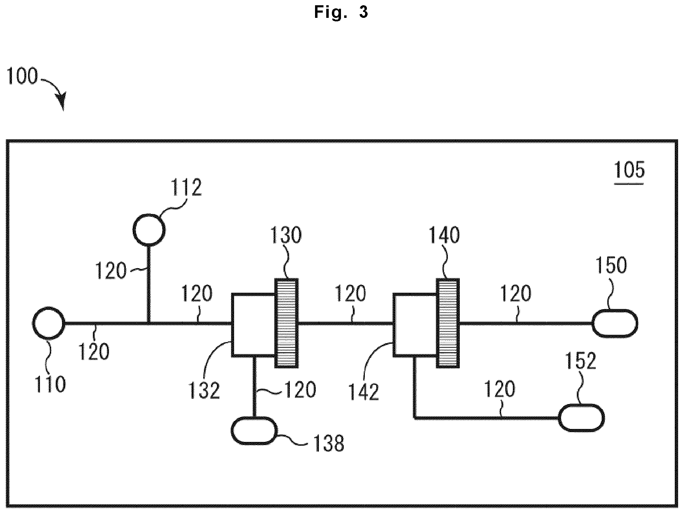

In the present invention, in order to ensure that the first component and the second component in the solution can pass through the second filter 130 and the first component can pass through the first filter 140, circulation flow channels 132 and 142 (which will be described in detail later, and see Figure 3) can be provided.

Examples of the combination of the first component, the second component and the third component are shown below.

**Table 2**

| | First component | Second component | Third component |
|---|---|---|---|
| (a) | Component to be used as analysis control | Component to be analyzed | Component other than the first component and the second component |
| (b) | Component to be analyzed | Component to be used as analysis control | Component other than the first component and the second component |
| (c) | Component to be used as analysis control | Component other than the first component and the third component | Component to be analyzed |
| (d) | Component other than the second component and the third component | Component to be used as analysis control | Component to be analyzed |
| (e) | Component to be analyzed | Component other than the first component and the third component | Component to be used as analysis control |
| (f) | Component other than the second component and the third component | Component to be analyzed | Component to be used as analysis control |

Examples of the respective components in the above-described aspects (a) to (f) are shown below.

### (1) Aspect (a)

First component (control component): group of low-molecular-weight molecules
Second component (component to be analyzed): biopolymer
Third component (component other than the first and second components): SVP (Sub visible particle: aggregate), residue such as cell debris, or coarse particle

### (2) Aspect (b)

First component (component to be analyzed): biopolymer
Second component (control component): polysaccharide, nucleic acid, synthetic polymer or the like (utilized as buffering agent or stabilizer)
Third component (component other than the first and second components): SVP, residue such as cell debris, or coarse particle

### (3) Aspect (c)

First component (control component): biopolymer (non-assembly, not used as measurement target in Aspect (c))
   In this regard, a "non-assembly" refers to a matter in which no assembly is formed with biopolymers by physical or chemical polymerization, association, aggregation or the like. The same applies to the following.
Second component (component other than the first and third components): SVP, residue such as cell debris, or coarse particle
Third component (component to be analyzed): assembly of biopolymers (physical/chemical assembly, measurement target)

In this regard, an "assembly" of biopolymers refers to a matter in which an assembly is formed with biopolymers by physical or chemical polymerization, association, aggregation or the like. The same applies to the following.

### (4) Aspect (d)

First component (component other than the second and third components): liquefied gas, supercritical fluid, low molecular weight carrier or the like
Second component (control component): polysaccharide, nucleic acid, synthetic polymer or the like
Third component (component to be analyzed): assembly of biopolymers (physical/chemical assembly, measurement target)

### (5) Aspect (e)

First component (component to be analyzed): biopolymer
Second component (component other than the first and third components): SVP, residue such as cell debris, or coarse particle
Third component (control component): polysaccharide, nucleic acid, synthetic polymer or the like

### (6) Aspect (f)

First component (component other than the second and third components): liquefied gas, supercritical fluid, low molecular weight carrier or the like
Second component (component to be analyzed): biopolymer
Third component (control component): polysaccharide, nucleic acid, synthetic polymer or the like

Regarding the biopolymer in the present invention, there is a case where substances derived from the biopolymer aggregate to form an aggregate. When this aggregate has a size that is larger than that of the pore of the second filter 130, the aggregate cannot pass through the second filter 130. In the present invention, such an aggregate that is formed with a plurality of biopolymers and cannot pass through the second filter can be handled as the third component (Aspects (c) and (d)).

The microchannel device of the present invention will be more specifically described below.

Figure 3 is a schematic configuration diagram of a microchannel device 100 according to one embodiment of the present invention and describes details of the aspect shown in Figure 1B. Hereinafter, the aspect of Figure 1B will be described, but the aspect in which the second filter, the third flow channel 120d and the third storage section are not provided (Figure 1A) can also be easily understood by those skilled in the art.

The microchannel device 100 is a device, wherein flow channels, filters, an inlet, an outlet, storage sections, etc. are formed inside a plate-like base material 105 by utilizing a microfabrication technology such as MEMS (Microelectromechanical Systems) technology, cutting work, photolithography, laser processing technology or the like. Note that it is possible to form flow channels, etc. on the surface of the base material 105 by utilizing a microfabrication technology such as MEMS technology, cutting work, photolithography, laser processing technology or the like, followed by bonding another base material 105 to the surface thereof, thereby providing a structure in which flow channels are formed inside a material obtained by bonding the two base materials 105 together (such a material is also referred to as "base material 105"). It is also possible to directly form flow channels, etc. inside the base material 105 by means of laser beam or the like.

The material of the base material 105 of the microchannel device 100 is silicon, glass, or plastic. As the material of the base material 105, various plastic materials can be selected and are suitably selected in consideration of biological compounds, other components, etc. Examples of plastic materials include, but are not limited to, polycarbonate, polyethylene, polyvinyl chloride, polystyrene, polypropylene, polyester, polymethyl methacrylate, polyvinyl acetate, vinyl-acetate copolymer, styrene-methyl methacrylate copolymer, acrylonitrile-styrene copolymer, acrylonitrile-butadiene-styrene copolymer, nylon, polymethylpentene, silicon resin, amino resin, polysulfone, polyethersulfone, polyetherimide, fluororesin, and polyimide. Further, additives such as a pigment, a dye, an antioxidant and a flame retardant may be suitably mixed with these plastic materials.

The microchannel device 100 has a sample inlet 110, a buffer inlet 112, a second filter 130, a second circulation flow channel 132, a molecular cut-off filter 134, a first filter 140, a first circulation flow channel 142, a first component storage section 150 for storing the first component, and a second component storage section 152 for storing a component to be analyzed. These are connected to each other via a flow channel 120. In one aspect of the present invention, the flow channel 120 is formed to have a tunnel-like shape.

The sample inlet 110 is an opening for introducing a solution containing a biopolymer as a target and other components into the flow channel 120. The biopolymer may be a single component or a complex in which a plurality of components are bonded, and it is, for example, a protein, lipid, sugar chain, nucleic acid, polymeric peptide, viral capsid, collagen, cyclofibroin, gelatin, starch, or cellulose.

The buffer inlet 112 is an opening for introducing a sending buffer solution into the flow channel 120. The sending buffer solution is a liquid for supporting a solution to flow through the flow channel 120, etc. In the case where the solution can sufficiently flow through the flow channel 120, etc. in the microchannel device 100 without the sending buffer solution, there is no need to use the sending buffer solution, and therefore it is not required to provide the buffer inlet 112 to the microchannel device 100. The sending buffer solution is introduced in a manner such that air exists between the solution and the buffer solution so that the sending buffer solution does not mix with the solution, and the sending buffer solution pushes the solution via air.

The solution injected from the sample inlet 110 passes through the flow channel 120 and flows into the second circulation flow channel 132, and while the solution circulates through the second circulation flow channel 132, the solution is filtered by the second filter 130 connected to the second circulation flow channel 132. Since the solution is gradually filtered by the second filter while circulating through the second circulation flow channel 132, clogging of the filter is reduced.

The second filter 130 has a molecular cut-off filter 134. The molecular cut-off filter 134 does not allow a component having a molecular weight (mass) equal to or larger than a second threshold contained in the solution to pass through, and allows the third component 180 having a molecular weight (mass) smaller than the second threshold contained in the solution to pass through. The second threshold is set so that the first component 160 and the second component 170 can pass through but the third component 180 having a molecular weight (mass) larger than those of the first component 160 and the second component 170 cannot pass through. The second threshold is, for example, 300 kDa, 100 kDa, 50 kDa, or the like. Instead of the molecular weight, a filter having a predetermined pore size (e.g., a filtration membrane of 0.45 µm or 0.2 µm) for removing the third component having a large size may also be used.

The components that pass through the second filter 130 (the first component and the second component) flow through the flow channel 120 to the first circulation flow channel 142 and the first filter 140. Meanwhile, the third component 180 that cannot pass through the second filter 130 flows into the third storage section 138 to be subjected to recirculation, measurement, or discarding. Between the second circulation flow channel 132 and the third storage section 138, a valve (not shown) can be provided. By opening the valve when it is confirmed that a sufficient amount of the solution has passed through the second filter 130 (or after a predetermined amount of time), the solution flowing through the second circulation flow channel 132 is stored in the third storage section 138. The solution stored in the third storage section 138 may be recirculated by providing a flow channel (not shown) connected between the third storage section 138 and the sample inlet 110.

The solution containing the component that passes through the second filter 130 passes through the flow channel 120 and flows into the first circulation flow channel 142, and while the solution circulates through the first circulation flow channel 142, the solution is filtered by the first filter 140 connected to the first circulation flow channel 142. The first filter 140 has a molecular cut-off filter 144. The molecular cut-off filter 144 does not allow a component having a molecular weight (mass) equal to or larger than a first threshold contained in the solution to pass through, and allows a component having a molecular weight (mass) smaller than the first threshold contained in the solution to pass through. The first threshold is set so that the first component 160 and the second component 170 can pass through but the third component 180 having a molecular weight (mass) larger than those of the first component 160 and the second component 170 cannot pass through.

The first component 160 that passes through the first filter 140 flows through the flow channel 120 to the first storage section 150 and is stored in the first storage section 150. Meanwhile, the second component 170 that cannot pass through the first filter 140 circulates through the first circulation flow channel 142 and is then sent to the second storage section 152 via the flow channel 120. Between the first circulation flow channel 142 and the second storage section 152, a valve (not shown) can be provided. By opening the valve when it is confirmed that a sufficient amount of the solution has passed through the first filter 140 or after a predetermined amount of time, the solution circulating through the first circulation flow channel 142 flows into the second storage section 152 and is stored in the second storage section 152.

In the present invention, the first storage section and the second storage section may be a section for storing the flowing solution or may be an outlet connected to another device such as a measuring device.

The solution stored in the first storage section 150 is a solution containing a component having a molecular weight (mass) smaller than the first threshold. The solution stored in the second storage section 152 is a solution containing a component having a molecular weight (mass) equal to or larger than the first threshold. Further, it can also be said that the solution stored in the second storage section 152 is a solution containing a component having a molecular weight (mass) that is smaller than the second threshold and equal to or larger than the first threshold.

The first storage section 150 and the second storage section 152 may be directly connected to another measuring device (not shown). Said another measuring device connected to the first storage section 150 and the second storage section 152 is, for example, an X-ray diffractometer, and the measurement for identifying the structure of a biopolymer as a target is performed by means of small-angle X-ray scattering (SAXS), medium-angle X-ray scattering (MAXS) or the like. For example, the solution stored in the first storage section 150 and the solution stored in the second storage section 152 can be used as a sample (a solution containing a biopolymer as a target) and a control (for comparison), or as a control and a sample, respectively. By measuring both the solutions with another measuring device and taking the difference between them, a measurement value caused by the biopolymer as the target can be obtained more accurately than before.

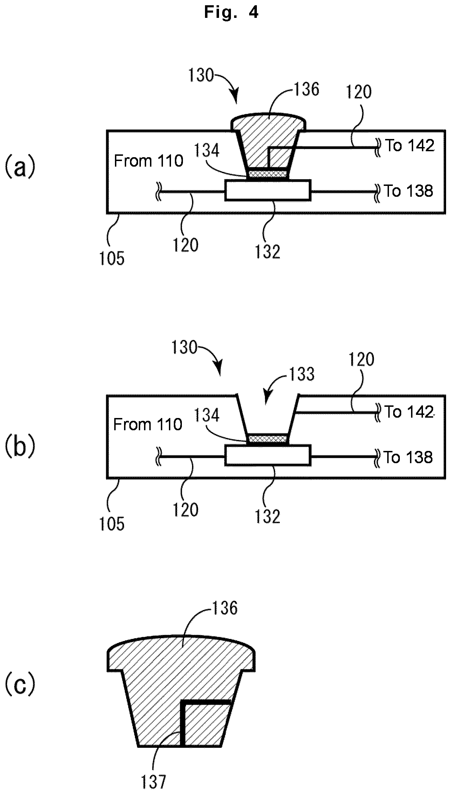

Figure 4(a) is a schematic cross sectional view of the second circulation flow channel 132 and the second filter 130 of the microchannel device 100. Figure 4(b) is a schematic cross sectional view showing a state in which a sealing member 136 is removed from the second filter 130. Figure 4(c) is a schematic view of the sealing member 136.

As shown in Figure 4(a), the flow channel 120, which is formed in the base material 105 and connected to the sample inlet 110, is connected to the second circulation flow channel 132 formed in the base material 105, and the second circulation flow channel 132 is in contact with the molecular cut-off filter 134.

In the base material 105, a concave portion 133 that is open to the upper portion is formed so that the molecular cut-off filter 134 can be replaced, and the molecular cut-off filter 134 is arranged in the concave portion 133 (Figure 4(b)). The molecular cut-off filter 134 is sealed from above with the sealing member 136 to prevent the solution from leaking from the concave portion 133 (Figure 4(a)).

The second circulation flow channel 132 is in contact with the molecular cut-off filter 134, and the solution circulating through the second circulation flow channel 132 is gradually filtered by the molecular cut-off filter 134 while circulating. A component with a value equal to or larger than the second threshold in the sample solution continues to circulate through the second circulation flow channel 132, while a component with a value smaller than the second threshold passes through the molecular cut-off filter 134.

A flow channel 137 is formed in the sealing member 136, and the flow channel 137 is connected to the flow channel 120 connected to first circulation flow channel 142. The solution that passes through the molecular cut-off filter 134 flows through the flow channel 137 to the first circulation flow channel 142. As described above, the solution circulating through the second circulation flow channel 132 can be taken out from the third storage section 138 according to the operation of the valve (not shown). Further, to the second storage section 152, both the component (group) with a value equal to or larger than the second threshold and the component (group) with a value smaller than the second threshold flow.

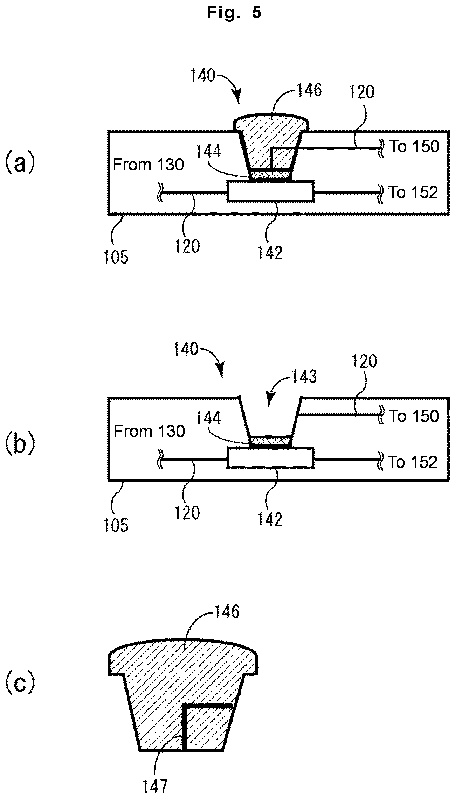

The constitution of the first filter 140 is similar to the constitution of the second filter 130. Figure 5(a) is a schematic cross sectional view of the first circulation flow channel 142 and the first filter 140 of the microchannel device 100. Figure 5(b) is a schematic cross sectional view showing a state in which a sealing member 146 is removed from the first filter 140. Figure 5(c) is a schematic view of the sealing member 146.

As shown in Figure 5(a), the flow channel 120, which is formed in the base material 105 and connected to the second filter 130, is connected to the first circulation flow channel 142 formed in the base material 105, and the first circulation flow channel 142 is in contact with the molecular cut-off filter 144.

In the base material 105, a concave portion 143 that is open to the upper portion is formed so that the molecular cut-off filter 144 can be replaced, and the molecular cut-off filter 144 is arranged in the concave portion 143 (Figure 5(b)). The molecular cut-off filter 144 is sealed from above with the sealing member 146 to prevent the solution from leaking from the concave portion 143 (Figure 5(a)). The first circulation flow channel 142 is in contact with the molecular cut-off filter 144, and the solution circulating through the first circulation flow channel 142 is gradually filtered by the molecular cut-off filter 144 while circulating. A component with a value equal to or larger than the first threshold in the sample solution continues to circulate through the first circulation flow channel 142, while a component with a value smaller than the first threshold passes through the molecular cut-off filter 144.

A flow channel 147 is formed in the sealing member 146, and the flow channel 147 is connected to the flow channel 120 connected to first storage section 150. The solution that passes through the molecular cut-off filter 144 flows through the flow channel 147 to the first storage section 150 and is stored therein. As described above, the solution circulating through the first circulation flow channel 142 flows to the second storage section 152 and is stored therein according to the operation of the valve (not shown). Further, the component (group) with a value smaller than the first threshold also flows to the second storage section 152.

Conventionally, a control solution is prepared by separately preparing components other than a biopolymer as a target and blending them. However, by using the microchannel device 100 according to one embodiment of the present invention, a sample containing a biopolymer as a target can be separated into a solution to be measured (containing the biopolymer as the target) and a control solution. When the biopolymer as the target is evaluated by measuring these separated solutions with another measuring device (for example, a device for analyzing the structure of the biopolymer using medium-angle X-ray scattering) and taking the difference between the measurement value of the solution to be measured (containing the biopolymer as the target) and the measurement value of the control solution, a more accurate measurement value can be obtained.

### EXAMPLES

Hereinafter, the present invention will be more specifically described by way of examples.

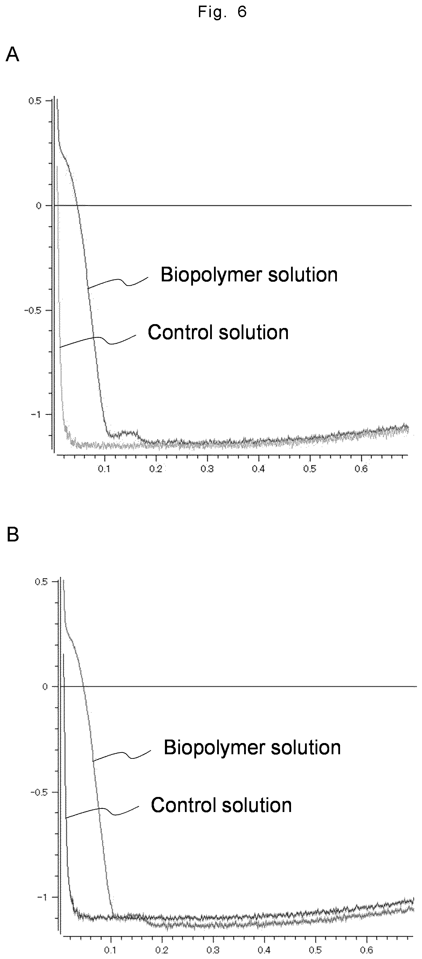

Figure 6 shows graphs showing results obtained by separation into a biopolymer as a target and a control component using a microchannel device according to one embodiment of the present invention and measurement of X-ray diffraction data of both of them.

Using glucose isomerase as the biopolymer to be analyzed, and using a buffer containing 0.1 M HEPES-Na (pH 7.0) and 10% glycerol, the scattering profile was measured with an MAXS device.

In Figure 6, A is a result obtained when using the microchannel device of the present invention, and it can be used as a difference profile. Meanwhile, B is a result obtained by the measurement according to the conventional method, and the scattering intensity of the control solution is higher than the scattering intensity of the biopolymer solution in the small-angle to medium-angle region. Therefore, the difference profile in this region is negative and cannot be used for analysis.

The sizes, materials, shapes, relative positions of constituents, etc. described in the embodiments above are optional and may be changed depending on the structure of the device to which the present invention is applied or various conditions. Further, the present invention is not limited to the specifically described embodiments above.

### EXPLANATIONS OF LETTERS OR NUMERALS

- 100: microchannel device
- 105: base material
- 110: inlet
- 120: flow channel
- 130: second filter
- 132: second circulation flow channel
- 133: second concave portion
- 134: molecular cut-off filter
- 136: second sealing member
- 137: flow channel
- 138: third storage section
- 140: first filter
- 142: first circulation flow channel
- 144: molecular cut-off filter
- 146: first sealing member
- 147: flow channel
- 150: first storage section
- 152: second storage section

## Claims

1. A microchannel device that separates a sample solution containing a first component and a second component having a larger size than the first component, into a solution containing the second component and a solution containing the first component but not the second component, and the microchannel device has:
a filter (first filter) with a pore size that allows the first component to pass through but does not allow the second component to pass through;
a first flow channel connected to a first reservoir section for storing the solution that passes through the first filter; and
a second flow channel connected to a second reservoir section for storing a solution that does not pass through the first filter.

2. The device according to claim 1, wherein the first component is a component used as an analysis control and the second component is a component to be analyzed.

3. The device according to claim 1, wherein the first component is a component to be analyzed and the second component is a component used as an analysis control.

4. The microchannel device according to claim 1, wherein the sample solution further contains a third component which is a component other than the first component and the second component and has a size larger than those of the first component and the second component, and wherein the microchannel device separates the sample solution into a solution containing the third component and a solution containing the first component and the second component but not containing the third component, the microchannel device further comprising:
a filter (second filter) having a pore size that allows the first component and the second component to pass through but does not allow the third component to pass through; and
a third flow channel connected to a third storage section for storing a solution that does not pass through the second filter,
wherein the second filter is arranged between the second flow channel and the third flow channel.

5. The device according to claim 4, wherein the first component, the second component and the third component are components combined according to any one of (a) to (f) below:
**Table 3**
| | First component | Second component | Third component |
|---|---|---|---|
| (a) | Component to be used as analysis control | Component to be analyzed | Component other than the first component and the second component |
| (b) | Component to be analyzed | Component to be used as analysis control | Component other than the first component and the second component |
| (c) | Component to be used as analysis control | Component other than the first component and the third component | Component to be analyzed |
| (d) | Component other than the second component and the third component | Component to be used as analysis control | Component to be analyzed |
| (e) | Component to be analyzed | Component other than the first component and the third component | Component to be used as analysis control |
| (f) | Component other than the second component and the third component | Component to be analyzed | Component to be used as analysis control |

6. The microchannel device according to claim 1, which further comprises a circulation flow channel (first circulation flow channel) that is connected to the first filter and stores and circulates the sample solution containing the first component and the second component.

7. The microchannel device according to claim 4, which further comprises a circulation flow channel (second circulation flow channel) that is connected to the second filter and stores and circulates the sample solution containing the first component, the second component and the third component.

8. The microchannel device according to claim 1, wherein the second component consists of a biopolymer and the first component consists of a low-molecular-weight substance which adjusts an environment of the biopolymer.

9. The microchannel device according to claim 4, wherein the second component consists of a biopolymer, the first component consists of a low-molecular-weight substance which adjusts an environment of the biopolymer, and the third component is an aggregate, a cell residue or a coarse particle.

10. The microchannel device according to claim 4, wherein the second filter has pores having a size that is smaller than that of the third component and larger than those of the first component and the second component.

11. A method for flowing a sample solution through the microchannel device according to claim 1 to separate the sample solution into a solution containing the second component and a solution containing the first component but not containing the second component.

12. The method for flowing a sample solution through the microchannel device according to claim 4 to separate the sample solution into a solution containing the third component and a solution containing the first component and the second component but not containing the third component, followed by separating into a solution containing the second component and a solution containing the first component but not containing the second component.
